# EUROPÄISCHE PATENTANMELDUNG

(11) **EP 3 760 132 A1**
(43) Veröffentlichungstag der Anmeldung: **06.01.2021**
(21) Anmeldenummer: 19184510.6
(22) Anmeldetag: 04.07.2019
(51) Int. Cl.: A61B 17/02, A61B 8/08

(54) **VORRICHTUNG ZUM HALTEN VON UNTERSCHIEDLICHEN ORGANEN IM ABDOMINALBEREICH**

(71) Anmelder: Q Medical International AG, 8260 Stein am Rhein (CH)
(72) Erfinder: Desombre, Rainer, 40667 Meerbusch (DE)
(74) Vertreter: dompatent von Kreisler Selting Werner - Partnerschaft von Patent- und Rechtsanwälten mbB

(57) **Zusammenfassung**

Bei einer Vorrichtung (1) zum Halten von unterschiedlichen Organen im Abdominalbereich, mit einem Haltekörper (2) der die Organstrukturen hält, wobei an dem Haltekörper (2) Halteelemente (20) angeordnet sind, die im Abdominalbereich befestigbar sind, ist vorgesehen, dass der Haltekörper (2) zumindest ein erstes ringförmiges Element (4) aufweist, an dem zumindest drei Halteöffnungen (7) angeordnet sind, an denen jeweils ein Halteelement (20) befestigt ist.

## Beschreibung

Die Erfindung betrifft eine Vorrichtung zum Halten von unterschiedlichen Organen im Abdominalbereich nach dem Oberbegriff des Anspruchs 1.

Bei Operationen im Abdominalbereich müssen Organe, an denen die jeweilige Operation nicht durchgeführt werden soll, aus dem Operationsfeld gehalten werden. Diese Organe müssen beispielsweise von zusätzlichem medizinischem Personal durchgehend gehalten werden. Alternativ werden schnurähnlich Element im Abdominalbereich festgenäht, die die Organe aus dem Operationsfeld halten.

Die Aufgabe der vorliegenden Erfindung ist es daher eine Vorrichtung zu schaffen, bei der bei Operationen das Operationsfeld auf besonders einfache Art und Weise zuverlässig freigehalten werden kann.

Zur Lösung dieser Aufgabe dienen die Merkmale des Anspruchs 1.

Die Vorrichtung zum Halten von unterschiedlichen Organen im Abdominalbereich gemäß der vorliegenden Erfindung weist folgende Merkmale auf:
- einen Haltekörper der die Organstrukturen hält, wobei
- an dem Haltekörper Halteelemente angeordnet sind, die im Abdominalbereich befestigbar sind.

Die Erfindung sieht in vorteilhafter Weise vor, dass der Haltekörper zumindest ein erstes ringförmiges Element aufweist, an dem zumindest drei Halteöffnungen vorgesehen sind, an denen jeweils ein Halteelement befestigt ist.

Die vorliegende Erfindung hat den Vorteil, dass das ringfömige Element die Organstrukturen besonders effektiv aus dem Operationsfeld halten kann. Auch die Halteelemente können einfach an dem Haltekörper befestigt werden. Die erfindungsgemäße Vorrichtung kann auch als Rückhaltesystem für jegliche Organ- und oder Gewebestrukturen bezeichnet werden.

Der Haltekörper kann weitere ringförmige Elemente aufweisen, die einen kleineren Durchmesser aufweisen als das erste ringförmige Element und die vorzugsweise mit dem ersten ringförmigen Element über Stege verbunden sind.

Es können mindestens zwei weitere ringförmige Elemente vorgesehen sind. Die Stege, die die ringförmigen Elemente verbinden, können vorzugsweise radial verlaufen.

Das erste und die weiteren ringförmigen Elemente können alle konzentrisch zueinander angeordnet sein. Konzentrisch bedeutet, dass das erste und die weiteren ringförmigen Elemente denselben Mittelpunkt aufweisen.

Es kann ein kreisförmiges Element angeordnet sein, das in der Mitte der ringförmigen Elemente angeordnet ist und ebenfalls konzentrisch zu den ringförmigen Elementen angeordnet sein. Der Mittelpunkt des kreisförmigen Elements bildet somit den Mittelpunkt der ringförmigen Elemente. Das kreisförmige Element ist ebenfalls vorzugsweise über Stege mit den weiteren ringförmigen Elementen verbunden sein. Auch diese Stege können vorzugsweise radial verlaufen.

Die Halteöffnungen können mittels Halteösen verstärkt sein, wobei die Halteösen vorzugsweise einstückig mit dem Haltekörper ausgebildet sind. Halteöse ist ein element, dass um die Öffnung angeordnet ist und verhindert, dass die Öffnung einreißt. Die Halteöse kann von dem Haltekörper abstehen. Die Halteöse kann jedoch das gleich Material aufweisen wie der Haltekörper.

Vorzugsweise weisen die Halteösen eine Steifigkeit auf, die höher ist als die Steifigkeit des ersten ringförmigen Elements.

Mit der Steifigkeit ist in bei der vorliegenden Erfindung die Dehnsteifigkeit gemeint. Die Steifigkeit ist eine Größe, die den Widerstand eines Körpers gegen elastische Verformung durch eine Kraft beschreibt. Die Steifigkeit hängt von den elastischen Eigenschaften des Werkstoffs (dem Elastizitätsmodul) und auch von der Geometrie des Bauteils ab.

Wenn die Halteöse eine höhere Steifigkeit als der Haltekörper aufweisen soll, so kann die Halteöse ein anders Material aufweisen, das weniger elastisch ist, oder die Halteöse weist eine solche Geometrie auf, dass die Halteösen eine höhere Steifigkeit aufweist als der Haltekörper. Die Steifigkeit kann beispielsweise mit Dehnversuchen ermittelt bzw. verglichen werden.

Die Halteöffnungen sind vorzugsweise nur an dem ersten ringförmigen Element angeordnet.

Der Haltekörper kann elastisch verformbar sein. Dies bedeutet, dass der Haltekörper ein Material aufweist, das elastisch verformbar ist. Elastische Verformbarkeit ist die Eigenschaft eines Körpers oder Materials, unter Krafteinwirkung seine Form zu verändern und bei Wegfall der einwirkenden Kraft in die Ursprungsform zurückzukehren. Der vorliegende Haltekörper kann vorzugsweise um mindestens 200 %, insbesondere um mindestens 300% insbesondere um mindestens 500 % und besonders bevorzugt um mindestens 700% bei Einwirkung einer Kraft die Länge verändern und kann dann nach Wegfall der Kraft wieder in die Ursprungsform zurückkehren.

Die Steifigkeit des ersten ringförmigen Elements kann höher sein als die Steifigkeit der weiteren ringförmigen Elemente. Dies hat den Vorteil, dass das erste ringförmige Element vorgibt, wie weit die Hakenelemente voneinander angeordnet sein können und welche Kräfte an diesen Haltelemente angreifen. Die weiteren ringförmigen Elemente tragen lediglich für das bessere Halten von Organen bei, so dass nichts zwischen dem ersten ringförmigen Element durchrutschen kann.

Die Halteelemente können Hakenelemente aufweisen oder Hakenelemente sein.

Dass das Hakenelement kann aus einem Material bestehen, das Titan aufweist.

Das Hakenelement kann zumindest zwei gebogene Bereiche aufweisen, wobei der erste gebogene Bereich das Halteelements in Abdominalbereich befestigbar ist, wobei der zweite gebogene Bereich an der Halteöffnung befestigbar ist. der erste gebogene Bereich das Halteelements kann auch eine Spitze aufweisen

Das Hakenlement kann einen Bereich aufweisen, in dem das Hakenelement eine erste und eine zweite Außenseite aufweisen, die einander gegenüberliegend angeordnet sind und jeweils flach ausgebildet sind.

Der Bereich, in dem die erste und die zweite Außenseite angeordnet sind, kann vorzugweise zwischen dem ersten gebogenen Bereich und dem zweiten gebogenen Bereich angeordnet sein.

Der erste und der zweite gebogene Bereich können zumindest in einer Ebene verlaufen, die vorzugsweise parallel zu der ersten und zweiten Außenseite verlaufen kann.

Der Haltekörper kann zumindest drei, vorzugsweise mindestens vier Halteöffnungen aufweisen.

Der Haltekörper kann aus einem Material bestehen, das Silikon aufweist. Auch die Halteösen können aus demselben Material bestehen.

Es können verschieden Vorrichtungen mit verschiedenen Haltekörpern unterschiedlicher Flexibilität bzw Steifigkeit hergestellt werden.

Der Haltekörper kann aus einem Material bestehen, das Barium Sulfat aufweist. Auf diese Weise kann der Haltekörper durch Röntgenaufnahmen leicht entdeckt werden.

Die Halteösen können in radialer Richtung nach Außen gegenüber dem ersten ringförmigen Element überstehen.

Die Hakenelemente können lösbar oder nicht lösbar sein.

Das erste ringförmige Element kann einen Querschnitt aufweisen, der größer ist als die jeweiligen Querschnitte der weiteren ringförmigen Elemente. Das erste ringförmige Element und die weiteren ringförmigen Elemente bestehen vorzugsweise aus dem gleichen Material. Aufgrund der unterschiedlichen Geometrie ist die Steifigkeit des ersten ringförmigen Elements jedoch höher als die Steifigkeit der weiteren ringförmigen Elemente.

Generell kann die Steifigkeit bzw. Flexibilität der unterschiedlichen ringförmigen Elemente unterschiedlich sein. Die Steifigkeit des ersten ringförmigen Elements muss nicht unbedingt höher sein als die Steifigkeit der anderen Elemente. Dies könnte auch anders herum sein.

Ringförmig bedeutet bei der vorliegenden Erfindung nicht zwangsweise rund. Das ringförmige Element kann auch eine eckige oder beliebige Form aufweisen. Ringförmig bedeutet lediglich, dass das Element kein Ende aufweist sondern umlaufend ausgebildet ist.

Im Folgenden wird unter Bezugnahme auf die Zeichnungen Ausführungsbeispiele der vorliegenden Erfindung näher erläutert.

Es zeigen schematisch:
- Fig. 1: eine Vorrichtung zum Halten von unterschiedlichen Organen im Abdominalbereich in der perspektivischen Ansicht,
- Fig. 2: eine Draufsicht auf die Vorrichtung,
- Fig. 3: eine Seitenansicht auf die Vorrichtung gemäß Fig. 2, und
- Fig. 4: zeigt ein Halteelement,
- Fig. 5: zeigt ein weiteres Halteelement

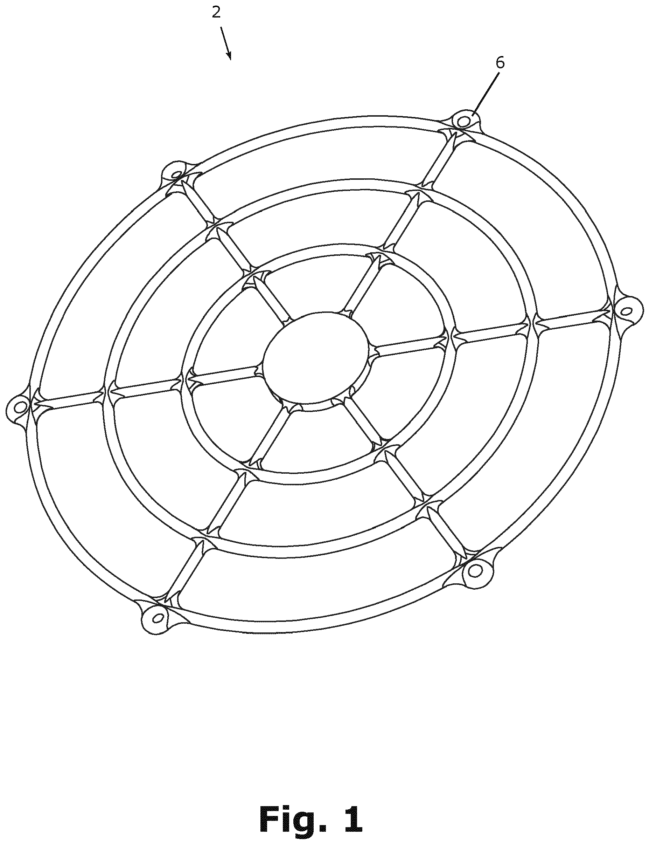

Fig. 1 zeigt eine Vorrichtung zum Halten von unterschiedlichen Organen im Abdominalbereich.

In Fig. 1, Fig. 2 und Fig. 3 ist ein Haltekörper 2 der Vorrichtung 1 dargestellt.

Die Vorrichtung 1 weist ferner Halteelemente 20 auf, die an dem Haltekörper 4 befestigt sind, die allerdings in den Fig. 1-3 nicht dargestellt sind.

Der Haltekörper 2 weist zumindest ein erstes ringförmiges Element 4 auf. An dem ersten ringförmigen Element 4 sind zumindest drei Halteöffnungen 7 angeordnet. In dem dargestellten Ausführungsbeispiel sind sechs Halteöffnungen 7 angeordnet.

An diesen Halteöffnungen 7 können die später noch genauer beschriebenen Halteelemente 20 befestigt sein. Der dargestellte Haltekörper 2 weist weitere ringförmige Elemente 8 und 10 auf, die jeweils einen kleineren Durchmesser d₂, d₃ aufweisen als das erste ringförmige Element 4 mit seinem Durchmesser d₁.

Die weiteren ringförmigen Elemente 8, 10 sind über Stege 14, 16 mit dem ersten ringförmigen Element 4 verbunden. Das zweite ringförmige Element 8 ist über den Steg 14 direkt mit dem ersten ringförmigen Element 4 verbunden. Das dritte ringförmige Element 10 ist über den Steg 16 und ferner über den Steg 14 mit dem ersten ringförmigen Element 10 verbunden. Alle Stege 14, 16 verlaufen in dargestellten Ausführungsbeispiel radial. Dies ist ein bevorzugtes Ausführungsbeispiel. Diese Stege könnten jedoch auch anders angeordnet sein. In dargestellten Ausführungsbeispiel ist ferner ein kreisförmiges Element 12 angeordnet. Das kreisförmige Element und das erste ringförmige Element sowie die weiteren ringförmigen Elemente sind konzentrisch zueinander angeordnet. Das kreisförmige Element 12 ist in der Mitte des ersten ringförmigen Elements 4 und der weiteren ringförmigen Elemente 8, 10 angeordnet.

Die Halteelemente 20 können in allen sechs Halteöffnungen 7 angeordnet sein. Sie sind jedoch mindestens in drei Halteöffnungen 7 angeordnet, beziehungsweise befestigt. Die Halteöffnungen 7 können mittels Halteösen 6 verstärkt sein. Dies bedeutet, dass die Halteösen 6 vorzugsweise eine höhere Steifigkeit aufweisen als das erste ringförmige Element 4. Die Halteösen 6 können einstückig mit dem Haltekörper 4 ausgebildet sein.

Die Halteösen können durch ein zusätzliches Material oder aufgrund der Geometrie eine höhere Steifigkeit aufweisen als das erste ringförmige Element 4.

Der Haltekörper 2 ist vorzugsweise elastisch verformbar. Die Steifigkeit des ersten ringförmigen Elements 4 ist vorzugsweise höher als die jeweiligen Steifigkeiten der weiteren ringförmigen Element 8, 10. Die Steifigkeit des ersten ringförmigen Elements 4 kann auch höher sein als die des kreisförmigen Elements 12.

Wenn die Halteelemente 20 nun an zumindest drei Halteöffnungen befestigt sind und in den Abdominalbereich eingehakt werden, kann aufgrund der elastischen Verformbarkeit des Haltekörpers 2, der Haltekörper 2 elastisch verformt und gespannt werden. Es können damit Organe aus einem Opertionsbereich gehalten werden.

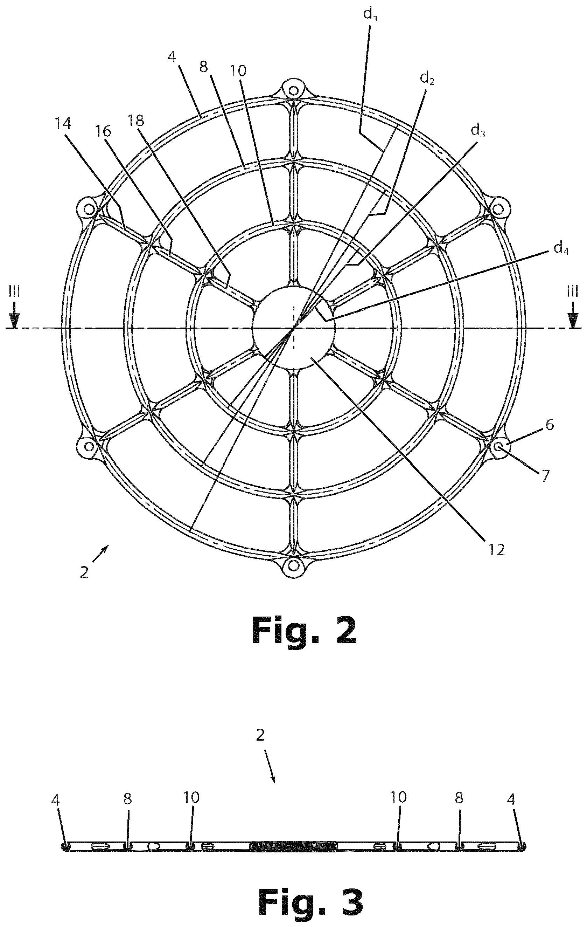

Der Haltekörper 2 muss keine runde Form aufweisen, sondern kann eine beliebige Form aufweisen. Generell bedeutet ringförmig nicht gleich rund. Das ringförmige Element 4 kann eine runde Form ausweisen, es kann jedoch auch eine beliebige andere beispielsweise eine eckige Form aufweisen. Ringförmig bedeutet lediglich, dass kein erstes oder zweites Ende vorhanden ist. Das ringförmige Element ist umlaufend so wie in Fig. 2 dargestellt.

Die Halteösen 6 stehen vorzugsweise, zumindest teilweise gegenüber dem ersten ring Element 4 in radialer Richtung nach Außen über.

Die Halteelemente 20 können lösbar oder auch nicht lösbar mit dem Haltekörper 2 verbunden sein.

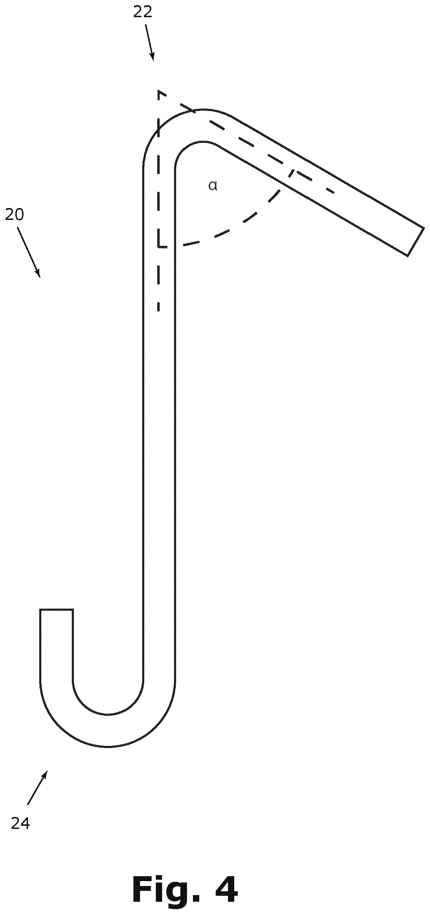

In Fig. 4 ist ein Halteelement 20 dargestellt. Das Halteelement 20, dass in Fig. 4 dargestellt ist, ist ein Hakenelement. Das Hakenelement weist einen ersten gebogenen Bereich 22 und einen zweiten gebogenen Bereich 24 auf. Der erste gebogene Bereich 22 kann derart geformt sein, dass er in den Abdominalbereich eingehakt werden kann. Er kann dafür beispielsweise eine Spitze aufweisen oder einen derart geringen Durchmesser, dass ein Einhaken in dieses Gewebe ohne Probleme ausgeführt werden kann. Es ist ein Winkel α dargestellt, mit dem der gebogen Bereich 22 gebogen ist. Dieser Winkel kann variieren.

Der zweite gebogene Bereich 24 kann so gebogen werden, dass er ohne Probleme in die Halteöffnung 7 eingeführt werden kann. Der gebogene Bereich 24 kann nach dem einführen in die Halteöffnung weiter gebogen werden, sodass ein Wiederherauslösen aus der Halteöffnung nicht möglich ist und das Hakenelement nicht verloren geht.

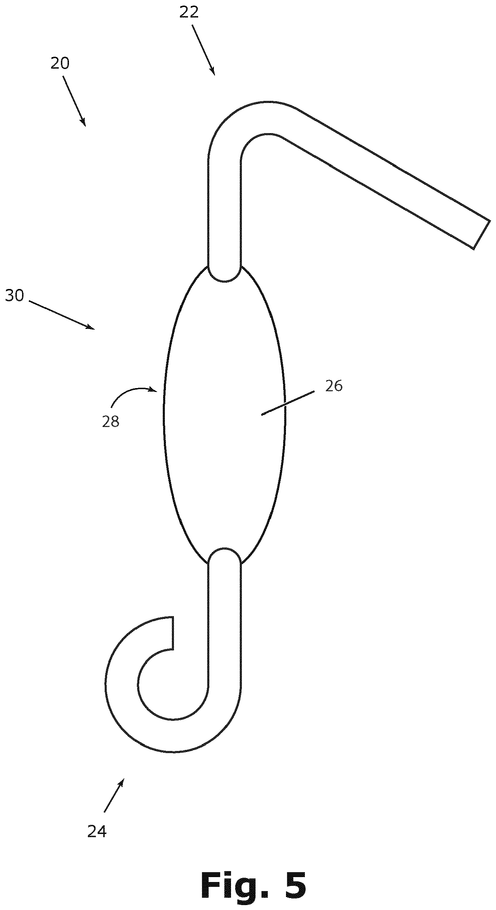

In Fig. 5 ist das Halteelement in einer alternativen Ausführungsform dargestellt. In Fig. 5 weist das Halteelement einen zusätzlichen Bereich 30 auf. In dem Bereich 30 weist das Halteelement 20 eine erste und eine zweite Außenseite 26, 28 auf.

Die erste und die zweite Außenseite sind parallel zueinander angeordnet und weisen in entgegengesetzte Richtungen.

Aufgrund der ersten oder der zweiten Außenseite 26, 28, die flach ausgebildet sind, kann das Hakenelement leicht mit einer Zange gegriffen werden. Dies ist besonders vorteilhaft, um bei einer Operation die Halteelemente 20 besonders gut im Abdominalbereich platzieren zu können. Der Bereich 30 ist vorzugsweise zwischen dem ersten gebogenen Bereich 22 und dem zweiten gebogenen Bereich 24 angeordnet. Der erste und der zweite gebogen Bereich 22, 24 verlaufen zumindest in einer Ebene, wobei die erste und die zweite Außenseite 28, 26 vorzugsweise parallel zu dieser Ebene verlaufen. Auf diese Weise stören der erste und der zweite gebogene Bereich 22, 24 beim Zugriff durch eine Zange nicht, wenn die Zange breiter ist als die erste und die zweite Außenfläche lang sind. Alternativ können die erste und die zweite Außenseite 26, 28 jedoch auch nicht parallel zu den gebogenen Bereichen 22, 24 ausgerichtet sein. In diesem Fall, muss lediglich eine Zange verwendet werden, die die Breite der Außenflächen 26, 28 nicht überschreitet. Flach bedeutet im Vorliegenden Fall jedoch auch, dass die Außenflächen auch eine geriffelte Oberfläche oder eine Struktur aufweisen können. Auf diese Weise würde eine Zange bei einem Zugriff auf die Außenflächen 26, 28 nicht verrutschen.

## Patentansprüche

1. Vorrichtung (1) zum Halten von unterschiedlichen Organen im Abdominalbereich, mit
- einem Haltekörper (2) der die Organstrukturen hält, wobei
- an dem Haltekörper (2) Halteelemente (20) angeordnet sind, die im Abdominalbereich befestigbar sind,
**dadurch gekennzeichnet,**
**dass** der Haltekörper (2) zumindest ein erstes ringförmiges Element (4) aufweist, an dem zumindest drei Halteöffnungen (7) angeordnet sind, an denen jeweils ein Halteelement (20) befestigt ist.

2. Vorrichtung nach Anspruch 1, **dadurch gekennzeichnet, dass** der Haltekörper (2) weitere ringförmige Elemente (8, 10) aufweist, die einen kleineren Durchmesser (d2, d3) aufweisen als das erste ringförmige Element und die vorzugsweise mit dem ersten ringförmigen Element (4) über Stege (14, 16, 18) verbunden sind.

3. Vorrichtung nach Anspruch 1 oder 2, **dadurch gekennzeichnet, dass** mindestens zwei weitere ringförmige Elemente (8, 10) vorgesehen sind.

4. Vorrichtung nach einem der Ansprüche 1 bis 3, **dadurch gekennzeichnet, dass** die Halteöffnungen (7) mittels Halteösen (6) verstärkt sind, wobei die Halteösen (6) vorzugsweise einstückig mit dem Haltekörper (4) ausgebildet sind.

5. Vorrichtung nach einem der Ansprüche 3 oder 4, **dadurch gekennzeichnet, dass** die Halteösen (6) eine Steifigkeit aufweisen, die höher ist als das erste ringförmige Element (4).

6. Vorrichtung nach einem der Ansprüche 1 bis 5, **dadurch gekennzeichnet, dass** der Haltekörper (2) elastisch verformbar ist.

7. Vorrichtung nach einem der Ansprüche 1 bis 6, **dadurch gekennzeichnet, dass** die Steifigkeit des ersten ringförmigen Elements (4) höher ist als die Steifigkeit der weiteren ringförmigen Elemente (8, 10).

8. Vorrichtung nach einem der Ansprüche 1 bis 7, **dadurch gekennzeichnet, dass** die Halteelemente (20) Hakenelemente aufweisen oder Hakenelemente sind.

9. Vorrichtung nach Anspruch 8, **dadurch gekennzeichnet, dass** das Hakenelement zumindest zwei gebogene Bereiche (22, 24) aufweist, wobei der erste gebogene Bereich (22) im Abdominalbereich befestigbar ist, wobei der zweite gebogene Bereich (24) an der Halteöffnung (7) befestigbar ist.

10. Vorrichtung nach Anspruch 8 oder 9, **dadurch gekennzeichnet, dass** das Hakenlement einen Bereich (26) aufweist, in der das Hakenelement eine erste und eine zweite Außenseite (28, 30) aufweist, die einander gegenüberliegend angeordnet sind und jeweils flach ausgebildet sind.

11. Vorrichtung nach einem der Ansprüche 1 bis 10, **dadurch gekennzeichnet, dass** der Haltekörper (2) aus einem Material besteht, das Silikon aufweist.

12. Vorrichtung nach Anspruch 11, **dadurch gekennzeichnet, dass** der Haltekörper (2) aus einem Material besteht, das Barium Sulfat aufweist.

13. Vorrichtung nach einem der Ansprüche 1 bis 12, **dadurch gekennzeichnet, dass** die Halteösen (6) nach außen gegenüber dem ersten ringförmigen Element (4) überstehen.

14. Vorrichtung nach einem der Ansprüche 1 bis 12, **dadurch gekennzeichnet, dass** Halteelemente (20) lösbar oder nicht lösbar sind.

15. Vorrichtung nach einem der Ansprüche 1 bis 12, **dadurch gekennzeichnet, dass** erste ringförmige Element (4) einen Querschnitt aufweist, der größer ist als der Querschnitt der weiteren ringförmigen Elemente (8, 10).
